# EUROPEAN PATENT APPLICATION

(11) **EP 4 595 957 A1**
(43) Date of publication of application: **06.08.2025**
(21) Application number: 23873190.5
(22) Date of filing: 27.09.2023
(51) Int. Cl.: A61K 31/351, A61P 9/00

(54) **PHARMACEUTICAL COMPOSITION COMPRISING ENAVOGLIFLOZIN FOR PREVENTING OR TREATING CARDIOVASCULAR AGING DISEASES**

(30) Priority: 29.09.2022 KR 20220123934
(71) Applicant: Daewoong Pharmaceutical Co., Ltd., Hwaseong-si, Gyenoggi-do 18623 (KR)
(72) Inventor: PARK, Sun-Hwa, Hwaseong-si Gyeonggi-do 18479 (KR); PARK, Joon Seok, Yongin-si, Gyeonggi-do 17000 (KR); CHOI, Ji-Soo, Seoul 06310 (KR); JI, Hye Young, Yongin-si Gyeonggi-do 17010 (KR); OAK, Min-Ho, Muan-gun Jeollanam-do 58568 (KR); KO, Ju-Young, Muan-gun Jeollanam-do 58580 (KR); SHIWAKOTI, Saugat, Muan-gun, Jeollanam-do Jeollanam-do 58553 (KR); BIKALPA, Dhakal, Muan-gun Jeollanam-do 58553 (KR)
(74) Representative: Plasseraud IP
(86) International application number: PCT/KR2023/014992
(87) International publication number: WO 2024/072079

(57) **Abstract**

The present invention relates to a pharmaceutical composition for use in preventing or treating cardiovascular aging diseases, comprising enavogliflozin as an active ingredient. Enavogliflozin of the present invention exhibits excellent effects in the prevention and treatment of endothelial dysfunction caused by vascular endothelial senescence, and cardiovascular aging diseases related thereto.

## Description

### [Technical Field]

The present invention relates to a pharmaceutical composition for use in preventing or treating a senescence-related cardiovascular disease, which comprises enavogliflozin as an active ingredient.

### [Background Art]

Senescence is a complex process of the interaction between physiological, psychological, and environmental changes and behavioral changes, which occur during the course of human life, and vascular senescence is a representative example of human senescence. In healthy blood vessels, vascular endothelium plays the most important role in regulating vascular homeostasis. Senescence-related cardiovascular diseases such as atherosclerosis rapidly increase due to endothelial dysfunction caused by vascular endothelial senescence.

Therefore, in order to derive innovative targets for use in preventing and treating senescence-related cardiovascular diseases, a strategy is needed to reduce the occurrence of cardiovascular diseases by inhibiting the occurrence of vascular senescence and the premature senescence process induced by specific factors.

The recent increase in environmental pollutants such as fine dust and microplastics has emerged as a major social issue, and research is being conducted on various aspects such as pollution, causes, and countermeasures. According to a report from the WHO, 72% of fine dust-related deaths are related to senescence-related cardiovascular diseases, and it is reported that the condition of the elderly and existing patients with senescence-related cardiovascular diseases is greatly accelerated. Microplastics are formed when plastic fragments are released into the environment and gradually break down into fine (≤5 mm) or nano (≤100 nm) particles, which are reported to be ingested and accumulated by other marine and freshwater organisms, fish, birds, and mammals, including humans. The possibility that the accumulation of microplastics in the body due to their exposure may cause oxidative stress and affect growth, reproduction, movement, behavior, and senescence (lifespan) has emerged, but the effects and mechanisms of long-term exposure to microplastics are currently unknown.

The sodium-glucose co-transporter (SGLT) is a glucose transport protein, with SGLT-1 mainly expressed in the small intestine, liver, and heart, and SGLT-2 mainly expressed in the kidneys. An SGLT-2 inhibitor is a new type of antidiabetic drug that reduces glucose resorption in the proximal nephron, thereby increasing glucose excretion through an insulin-independent mechanism. In addition, such an SGLT-2 inhibitor has been reported to reduce senescence-related cardiovascular disease mortality and hospitalization rates independently of glycemic control, and in nonclinical studies, it was observed that expression was increased in endothelial cells by high glucose and angiotensin II (an important factor involved in vascular senescence and endothelial dysfunction).

Accordingly, there is growing interest in the prevention or treatment of senescence-related cardiovascular diseases by SGLT-2 inhibitors.

### [Disclosure]

### [Technical Problem]

The present inventors have made diligent efforts to develop a composition for use in preventing or treating senescence-related cardiovascular diseases, confirming that enavogliflozin has an excellent effect on endothelial dysfunction occurring due to vascular endothelial senescence and cardiovascular senescence diseases related thereto. Thus, the present invention was completed.

Accordingly, the present invention is directed to providing a pharmaceutical composition for use in preventing or treating senescence-related cardiovascular diseases, which comprises enavogliflozin as an active ingredient.

The present invention is also directed to providing a method of preventing or treating senescence-related cardiovascular diseases in a subject in need thereof, which comprises administering a therapeutic effective amount of a pharmaceutical composition comprising enavogliflozin as an active ingredient to a subject in need thereof.

The present invention is also directed to providing a use of enavogliflozin to prepare a pharmaceutical composition for use in preventing or treating senescence-related cardiovascular diseases.

### [Technical Solution]

The present invention relates to a pharmaceutical composition for use in preventing or treating senescence-related cardiovascular diseases, which comprises enavogliflozin as an active ingredient, and a use of enavogliflozin to prevent or treat senescence-related cardiovascular diseases, and it was confirmed that the pharmaceutical composition according to the present invention is superior in preventing or treating cardiovascular senescence diseases.

Hereinafter, the present invention will be described in further detail.

One aspect of the present invention provides a pharmaceutical composition for use in preventing or treating senescence-related cardiovascular diseases, which comprises enavogliflozin as an active ingredient.

The term "enavogliflozin" used in the specification is, as a sodium-glucose cotransporter 2 (SGLT-2) inhibitor, a drug that selectively inhibits SGLT-2 involved in the resorption of glucose in the kidneys, thereby preventing glucose from being absorbed in the body and excreting it in the urine.

The "enavogliflozin" used herein may have the structure of Chemical Formula 1 below.

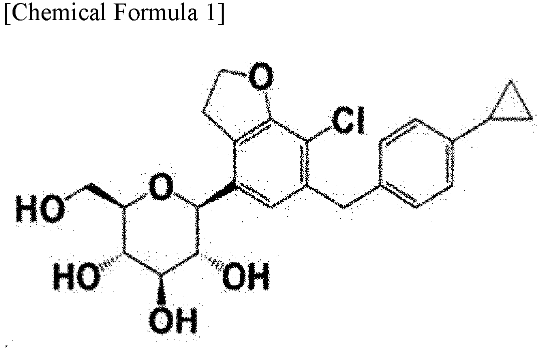

The term "prevention" used in the specification refers to all actions of inhibiting or delaying the occurrence of a senescence-related cardiovascular disease by the administration of the pharmaceutical composition according to the present invention.

The term "treatment" used in the specification refers to all actions involved in improving or beneficially changing a senescence-related cardiovascular disease by the administration of the pharmaceutical composition according to the present invention.

**In** the following examples, it was confirmed that nanoplastics are used to induce vascular endothelial senescence, and enavogliflozin can prevent or treat endothelial dysfunction caused by vascular endothelial senescence and senescence-related cardiovascular diseases related thereto.

Nanoplastics are tiny plastic fragments (≤100 nm) created during the decomposition of plastic products. The accumulation of nanoplastics in the body has been reported to cause oxidative stress and affect growth, reproduction, movement, behavior, and senescence (lifespan). In the following examples, it was confirmed that nanoplastics penetrate into porcine coronary artery endothelial cells to increase the activity of β-galactosidase (SA-β-Gal) as a histochemical marker of cell senescence, cause a decrease in endothelial cell proliferation, increase the expression of p53 and p21, which are cell senescence markers, and increase the expression of NADPH oxidase, which is known to cause cellular senescence and impairment of endothelial cell function by producing a peroxide in endothelial cells. These results demonstrate that the accumulation of nanoplastics causes vascular endothelial senescence and the resulting endothelial dysfunction. It is well known that an increase in p53 and p21 in endothelial cells leads to cellular senescence and a decrease in heart function. It has been reported that vascular dysfunction occurs due to the accumulation of these senescent cells, which causes atherosclerotic diseases such as aortic aneurysm, coronary artery disease, peripheral artery disease, and carotid artery disease (G. Katsuumi et al., Vascular Senescence in Cardiovascular and Metabolic Diseases Front Cardiovasc Med. 2018; 5: 18.). Cellular senescence impairs the essential role that the endothelium plays in maintaining vascular homeostasis, promoting the occurrence of endothelial dysfunction and senescence-related vascular diseases. It has been reported that SA-β-Gal is detected in senescent retinal vessels, atherosclerotic plaques of the aorta and coronary arteries, and adipose tissue obtained from obese subjects, and that p53 is elevated in patients with congestive heart failure or hypertrophic cardiomyopathy (YE Han & SY Kim, Endothelial senescence in vascular diseases: current understanding and future opportunities in senotherapeutics. Experimental & Molecular Medicine volume 55, pages 1-12 (2023)). Intense SA-β-Gal staining was observed in atherosclerotic lesions of coronary arteries obtained in autopsy from individuals with ischemic heart disease, and immunohistochemical analysis using anti-factor VIII antibodies demonstrated that the SA-β-Gal-stained cells were vascular endothelial cells. Vascular endothelial cell senescence increased atherosclerosis-associated ICAM-1 expression and decreased eNOS activity (T. Minamino et al., Endothelial cell senescence in human atherosclerosis: role of telomere in endothelial dysfunction. Circulation. 2002 Apr 2;105(13):1541-4.). In addition, when aortic endothelial cells were stained with SA-β-Gal in a low-density lipoprotein receptor^{-/-} (LDLR^{-/-}) mouse exposed to a high fat diet between 2 to 12 weeks as an atherosclerosis model, SA-β-Gal activity increased, and p53 expression increased. When comparing the external curvature (undisturbed flow) and internal curvature (disturbed flow) of the aorta, it was confirmed that senescent endothelial cells were accumulated in the internal curvature during the atherosclerotic process, which proved that senescence of endothelial cells was induced via the p53- p21 signaling pathway. These results suggest that the senescence of endothelial cells may be involved in the initiation and progression of atherosclerosis (C. M. Warboys et al., Disturbed Flow Promotes Endothelial Senescence via a p53-Dependent Pathway. Arterioscler Thromb Vasc Biol. 2014;34:985-995). It has been reported that senescent vascular cells accumulate in human atheroma tissue and exhibit various dysfunctional characteristics, and that cellular senescence may contribute to the pathogenesis of human atherosclerosis. It has been suggested that interactions between monocytes and vascular endothelial cells are enhanced by endothelial cell senescence, which may also promote atherosclerosis (T. Minamino et al., Vascular Cell Senescence. Circulation Research. 2007; 100: 15-26).

In the present invention, the vascular endothelial senescence experiment using nanoplastics was merely adopted as an experimental model to demonstrate that enavogliflozin is effective in preventing or treating endothelial dysfunction caused by vascular endothelial senescence, and the present invention is not limited to the prevention or treatment of senescence-related cardiovascular diseases caused by nanoplastics.

In the present invention, senescence-related cardiovascular diseases are associated with endothelial dysfunction caused by vascular endothelial senescence, and may be, for example, one or more selected from the group consisting of hypertrophic obstructive cardiomyopathy, severe obstructive coronary artery disease, aortic stenosis, hemodynamically significant aortic or mitral stenosis, aortic aneurysm, coronary artery disease, peripheral artery disease, and carotid artery disease, but the present invention is not limited thereto.

Another aspect of the present invention provides a method of preventing or treating senescence-related cardiovascular diseases in a subject in need thereof, which comprises administering a therapeutic effective amount of a pharmaceutical composition comprising enavogliflozin as an active ingredient to a subject in need thereof.

The pharmaceutical composition according to the present invention may further comprises a pharmaceutically acceptable carrier other than enavogliflozin of Chemical Formula 1 as an active ingredient or a pharmaceutically acceptable salt thereof, and may be formulated with a carrier.

The term "pharmaceutically acceptable carrier" used herein refers to a carrier or diluent that does not irritate the organism and does not inhibit the biological activity and properties of the administered compound. In the composition formulated as a liquid solution, a pharmaceutically acceptable carrier may comprise a saline solution, sterilized water, Ringer's solution, buffered saline, albumin injection, dextrose solution, maltodextrin solution, glycerol, ethanol, and a mixture of one or more components thereof, which are suitable for sterilization and biocompatible, and if needed, other common additives such as an antioxidant, a buffer, and a bacteriostatic agent may be added. In addition, by additionally adding a diluent, a dispersant, a surfactant, a binder, and a lubricant, the composition may be formulated into an injectable formulation such as an aqueous solution, a suspension, or an emulsion, a pill, a capsule, a granule, or a tablet.

In the present invention, the pharmaceutical composition may have a dosage form for oral administration, such as a tablet or a capsule. In one embodiment of the present invention, the pharmaceutical composition may be formulated as a tablet.

In the present invention, the pharmaceutical composition may have a dosage form for parenteral administration. A dosage form for parenteral administration, which comprises the composition of the present invention as an active ingredient, may be a form for injection such as subcutaneous injection, intravenous injection, or intramuscular injection, but the present invention is not limited thereto.

For formulation into an injectable form, the composition of the present invention may be mixed with water together with a stabilizing agent or a buffer to be prepared in a solution or suspension, which may be formulated for unit dosing in ampoules or vials.

Alternatively, the composition of the present invention may be formulated into various forms of parenteral administration, such as an eye drop, a microneedle, a patch, and a depot.

The pharmaceutical composition may be administered at a pharmaceutically effective amount. The term "pharmaceutically effective amount" used herein refers to an amount sufficient for treating a disease at a reasonable benefit/risk ratio applicable for medical treatment, and an effective dosage may be determined according to parameters including a type of a patient's disease, severity, drug activity, sensitivity to a drug, administration time, an administration route, an excretion rate, the duration of treatment and drugs simultaneously used, and other parameters well known in the medical field. The pharmaceutical composition of the present invention may be administered separately or in combination with other therapeutic agents, sequentially or simultaneously administered with a conventional therapeutic agent, or administered in a single or multiple dose(s). That is, the total effective amount of the composition of the present invention may be administered to a patient as a single dose, or may be administered by a fractionated treatment protocol in which multiple doses are administered over a long period of time. In consideration of all of the above-mentioned parameters, it is important to achieve the maximum effect with the minimum dose without side effects, and such a dose may be easily determined by one of ordinary skill in the art.

Th optimal daily dose of enavogliflozin determined during clinical trials is 0.1 to 0.5 mg.

When the pharmaceutical composition is formulated as a unit dosage form, the content of the active ingredient in the pharmaceutical composition may be 0.1 to 0.5 mg.

The pharmaceutical composition according to the present invention may be administered once to three times a day, for example, once a day. But the present invention is not limited thereto.

In the present invention, the dose of enavogliflozin that can be used to prevent or treat senescence-related cardiovascular diseases is not particularly limited, and may be appropriately adjusted depending on the severity of the disease, weight, age, sex, and the presence or absence of other complications.

The method of administering a pharmaceutical composition for use in preventing or treating senescence-related cardiovascular diseases uses a pharmaceutical composition for use in preventing or treating senescence-related cardiovascular diseases, and thus any overlapping content between these two is omitted to avoid excessive description in the specification.

Enavogliflozin used as an active ingredient in the present invention may be synthesized through the known related literatures. In the present invention, enavogliflozin may be crystalline or amorphous. For example, enavogliflozin may be crystalline form A, B, C, D, or E of enavogliflozin, or amorphous enavogliflozin, which is reported to have the following X-ray diffraction spectrum according to Korean Unexamined Patent Application Publication No. 2017-0142904 or Korean Patent Application No. 2022-0123673.

Crystalline form A: the crystalline form with an X-ray diffraction (XRD) spectrum having peaks at 2[θ] values selected from 6.2° ± 0.2°, 7.2° ± 0.2°, 8.8° ± 0.2°, 17.6° ± 0.2°, 19.0° ± 0.2°, 22.5° ± 0.2°, and 25.1° ± 0.2°

Crystalline form B: the crystalline form with an X-ray diffraction (XRD) spectrum having peaks at 2[θ] values selected from 7.0° ± 0.2°, 14.9° ± 0.2°, 17.7° ± 0.2°, 18.8° ± 0.2°, 20.6° ± 0.2°, 21.8° ± 0.2°, and 23.5° ± 0.2°

Crystalline form C: the crystalline form with an X-ray diffraction (XRD) spectrum having peaks at 2[θ] values selected from 5.6° ± 0.2°, 7.3° ± 0.2°, 15.7° ± 0.2°, 17.2° ± 0.2°, 18.9° ± 0.2°, 21.2° ± 0.2°, and 21.9° ± 0.2°

Crystalline form D: the crystalline form with an X-ray diffraction (XRD) spectrum having peaks at 2[θ] values selected from 5.5° ± 0.2°, 7.2° ± 0.2°, 15.3° ± 0.2°, 17.2° ± 0.2°, 17.6° ± 0.2°, 18.9° ± 0.2°, and 21.1° ± 0.2°

Crystalline form E: the crystalline form with an X-ray diffraction (XRD) spectrum having peaks at 2[θ] values selected from 4.93° ± 0.2°, 6.12° ± 0.2°, 7.43° ± 0.2°, 8.89° ± 0.2°, 9.74° ± 0.2°, 14.79° ± 0.2°, 15.79° ± 0.2°, 16.11° ± 0.2°, 19.79° ± 0.2°, and 22.83° ± 0.2°

Each of the crystalline forms A, B, C, and D is specified by the X-ray diffraction spectra having four or more, e.g., 4, 5, 6, 7, 8 or more, peaks at the presented 2[θ] values.

The pharmaceutical composition including enavogliflozin according to the present invention may have the composition of the pharmaceutical composition of PCT/KR2022/014640.

For example, the pharmaceutical composition of the present invention may be a pharmaceutical composition which comprises a compound of Chemical Formula 1 or a pharmaceutically acceptable salt thereof as an active ingredient, an excipient, a disintegrant, and a binder, and the average particle size of the compound of Chemical Formula 1 may be 15 µm or less.

In an exemplary embodiment of the present invention, the average particle size of enavogliflozin may be 15 µm or less, and preferably, 10 µm or less.

When the average particle size of enavogliflozin is greater than 15 µm, the 5-minute dissolution rate is very low at less than 40% of the total content of enavogliflozin, and the 30-minute dissolution rate is also less than 80% thereof, indicating that the final dissolution rate is inadequate.

When the micronization of drug particles is required, the drug particles may be crushed using a conventional mill that can micronize particles such as a Z-mill, a hammer mill, a ball mill, or a fluid energy mill. In addition, the drug particle size may be divided by a size classification method such as sieving using a sieve, or air current classification. Methods of controlling a desired particle size are well known in the art (e.g., refer to [Pharmaceutical dosage forms: volume 2, 2nd edition, Ed.: H. A. Lieberman, L. Lachman, J. B. Schwartz (Chapter 3: SIZE REDUCTION)]).

In the specification, the particle size of a drug is expressed on the basis of a particle size distribution such as D(X) = Y (where X and Y are positive numbers). D(X) = Y means that when the particle size distribution of a drug obtained by measuring the particle diameter of any drug in the preparation is represented by a cumulative curve, the particle diameter at the point where the particle size becomes X% while being accumulated in the order of decreasing particle size (% is calculated on the basis of a number, volume, or weight) is Y. For example, D(10) represents the particle diameter at the point where the particle size becomes 10% while being accumulated in the order of decreasing particle size, D(50) represents the particle diameter at the point where the particle size becomes 50% while being accumulated in the order of decreasing particle size, and D(90) represents the particle diameter at the point where the particle size becomes 90% while being accumulated in the order of decreasing particle size.

Whether the particle size distribution D(X) is based on number, volume, or weight as a percentage of the total cumulative particles depends on the method used to measure the particle size distribution. Methods for measuring particle size distribution and types of % associated therewith are known in the art. For example, when measuring particle size distribution by the well-known laser diffraction method, the X value in D(X) represents the percentage calculated by the average volume. It is well known by those of ordinary skill in the art that the particle size distribution measurement result obtained by a particular method can be correlated with those obtained from other techniques based on an experience obtained by routine experiments. For example, laser diffraction is sensitive to the particle volume and provides a volume average particle size, which is equivalent to a weight average particle size at constant density.

In the present invention, the measurement of the particle size distribution of drug particles may be performed using a commercially available device based on the laser diffraction/scattering method based on the Mie theory. For example, the particle size distribution is measured using a commercial device such as the Mastersizer laser diffraction device (Malvern Instruments). In this device, a helium-neon laser beam and a blue light-emitting diode are applied to the particles, causing scattering and thus producing a light scattering pattern on a detector. By interpreting the light scattering pattern according to the Mie theory, the particle diameter distribution is obtained. The measurement method can be either dry or wet.

For reference, in an example of the present invention, the particle size of the drug was measured by volume average particle size using laser diffraction.

In one embodiment of the present invention, the compound of Chemical Formula 1 may be comprised at less than 1 part by weight with respect to 100 parts by weight of the total pharmaceutical composition.

The appropriate daily dose of enavogliflozin identified during clinical trials is 0.1 to 0.5 mg, and when the pharmaceutical composition is formulated as a unit dosage form, the content of the active ingredient in the pharmaceutical composition may be 0.1 to 0.5 mg.

The pharmaceutical composition according to the present invention comprises pharmaceutically acceptable additives other than the compound of Chemical Formula 1 as an active ingredient.

The pharmaceutical composition of the present invention comprises an excipient, a disintegrant, and a binder.

Examples of excipients comprises lactose (including a hydrate), dextrin, mannitol, sorbitol, starch, microcrystalline cellulose (e.g., Celphere^{™}), silicified microcrystalline cellulose (e.g., Prosolv^{™}), calcium phosphate hydrate, anhydrous calcium phosphate, calcium carbonate, a saccharide, and a mixture thereof. In one embodiment of the present invention, a preferable excipient is microcrystalline cellulose.

Examples of disintegrants comprise crospovidone, croscarmellose sodium, sodium starch glycolate, and low-substituted hydroxypropyl cellulose. In one embodiment of the present invention, a preferable excipient is croscarmellose sodium.

Examples of binders comprise polyvinylpyrrolidone, povidone, gelatin, starch, sucrose, methylcellulose, ethylcellulose, hydroxyethylellulose, hydroxypropylcellulose, hydroxypropylalkylcelluloses (e.g., hydroxypropylmethylcellulose), and a mixture thereof. In one embodiment of the present invention, a preferable binder is hydroxypropylcellulose.

Examples of additives other than those listed above comprise a lubricant and a coloring agent.

The lubricant may be stearic acid, a stearate (e.g., magnesium stearate), light anhydrous silicic acid, talc, corn starch, carnauba wax, magnesium silicate, synthetic aluminum silicate, hydrogenated oil, white lead, titanium oxide, microcrystalline cellulose, Macrogol 4000 and 6000, isopropyl myristate, calcium hydrogen phosphate, or a mixture thereof.

In one embodiment of the present invention, the excipient may be comprised at 80 to 95 parts by weight with respect to 100 parts by weight of the total pharmaceutical composition.

In one embodiment of the present invention, the disintegrant may be comprised at 2 to 8 parts by weight with respect to 100 parts by weight of the total pharmaceutical composition. When the disintegrant is comprised at less than 2 parts by weight with respect to 100 parts by weight of the total pharmaceutical composition, the initial disintegration ability may be low and thus the dissolution rate may be delayed, affecting the Cmax in the body. In addition, when the disintegrant is comprised at more than 8 parts by weight with respect to 100 parts by weight of the total pharmaceutical composition, as the amount of disintegrant in the postmixed portion increases, the overall flowability of granules may decrease.

In one embodiment of the present invention, the binder may be comprised at 3 to 10 parts by weight with respect to 100 parts by weight of the total pharmaceutical composition. When the binder is comprised at less than 3 parts by weight with respect to 100 parts by weight of the total pharmaceutical composition, it may be difficult to form and maintain appropriate dry granules, affecting the maintenance of homogeneous dispersion of the main ingredient and the flowability of granules due to the generation of fine particles. In addition, when the binder is comprised at more than 10 parts by weight with respect to 100 parts by weight of the total pharmaceutical composition, granules with a strong binding strength are formed, affecting the solubility of the initially disintegrated granular particles during dissolution and also affecting the Cmax in the body.

The pharmaceutical composition according to the present invention may be an immediate-release formulation.

In one embodiment of the present invention, the pharmaceutical composition may have a dissolution rate after 5 minutes of 50% or more, and preferably, 60% or more of the total content of the active ingredient.

In one embodiment of the present invention, the pharmaceutical composition may have a dissolution rate after 15 minutes of 80% or more, and preferably, 85% or more of the total content of the active ingredient.

In one embodiment of the present invention, the pharmaceutical composition may have a dissolution rate after 30 minutes of 85% or more, and preferably, 90% or more of the total content of the active ingredient.

Since the dissolution rate of the active ingredient in the pharmaceutical composition affects the maximum blood concentration (Cmax) and the blood concentration-area under the blood concentration-time curve (AUC) when administering the drug, to achieve appropriate Cmax and AUC, it is important to adjust the dissolution rate of the pharmaceutical composition. Since enavogliflozin has a Tmax of 1 to 2 hours, the drug absorption rate in the stomach is considered important. The above dissolution rate was measured under the condition of the dissolved solution 1.2 in accordance with the dissolution test method 2 of the Korean Pharmacopoeia (paddle method). For specific conditions, refer to the following experimental examples.

The present invention also provides a pharmaceutical composition including a granule mixture of premixed granules, which comprise the compound of Chemical Formula 1 or a pharmaceutically acceptable salt thereof, and a postmixed portion.

During the research on the formulation of the compound of Chemical Formula 1, the present inventors confirmed that preparing granules and formulating them into tablets are advantageous in terms of drug content uniformity and the uniformity of dosage units.

In the pharmaceutical composition, the granules are prepared by mixing premixed granules and a postmixed portion.

The premixed granules may comprise the compound of Chemical Formula 1 or a pharmaceutically acceptable salt thereof, an excipient, a binder, and a lubricant.

In addition, the postmixed portion may comprise an excipient, a disintegrant, and a lubricant.

Descriptions of the excipient, the binder, the disintegrant, and the lubricant are the same as those described above, and are therefore omitted to avoid duplicate description.

In one embodiment of the present invention, each of the premixed granules and the postmixed portion may comprise an excipient. More specifically, each of the premixed granules and the postmixed portion may comprise microcrystalline cellulose as the excipient.

According to the following examples, the microcrystalline cellulose comprised in the premixed granules and the postmixed portion was found to affect the content uniformity of a drug depending on its particle size and bulk density.

In one embodiment of the present invention, the particle size of the microcrystalline cellulose in the premixed granules may be 130 µm or less, and preferably, 60 to 130 µm. The bulk density of the microcrystalline cellulose in the premixed granules may be 0.26 to 0.33. When the particle size and bulk density of the microcrystalline cellulose in the premixed granules are the same as the above conditions, it is possible to obtain a formulation with low standard deviation (SD) of content uniformity. When the particle size of the microcrystalline in the premixed granules is 130 µm or less, all of the content uniformity of the premixed granules, the content uniformity of the final granules, and the formulation showed good levels, and Carr's index values, which indicate the physical properties of the final granules, were also good, confirming that the flowability of the formulation was also excellent. On the other hand, when the particle size of the microcrystalline cellulose in the premixed granules exceeded 130 µm, neither the content uniformity of the premixed granules nor the content uniformity of the final granules was suitable due to large variations, and the formulation uniformity was also found to be poor.

Meanwhile, the particle size of the microcrystalline cellulose in the postmixed portion may be 130 µm or more, and preferably, 130 to 250 µm. The bulk density of the excipient in the postmixed portion may be 0.28 to 0.37. When the particle size of the microcrystalline cellulose in the postmixed portion is less than 130 µm, the Carr's index value, which indicates the physical properties of the final granules, was not appropriate and the granule flowability was poor.

When comparing the microcrystalline cellulose in the premixed granules and the microcrystalline cellulose in the postmixed portion, it is preferable that the particle size of the microcrystalline cellulose in the premixed granules be small, whereas it is preferable that the particle size of the microcrystalline cellulose in the postmixed portion be relatively larger than the particle size of the microcrystalline cellulose in the premixed granules.

According to the following examples, not only the particle sizes of the microcrystalline cellulose in the premixed granules and the microcrystalline cellulose in the postmixed portion but also the weight ratio of the excipient in the premixed granules and the excipient in the postmixed portion is confirmed to affect the content uniformity of a drug.

In one embodiment of the present invention, the weight ratio of the excipient in the premixed granules and the excipient in the postmixed portion may be 4:1 to 1:1. As the proportion of the microcrystalline cellulose in the postmixed portion increases, the flowability of the granules is improved, but the content variation increases, so it was found that it is preferable to adjust the weight ratio within the appropriate range.

Meanwhile, in the pharmaceutical composition according to the present invention, the binder may be one or more selected from the group consisting of hydroxypropyl cellulose, povidone, copovidone, and hypromellose.

In one embodiment of the present invention, the binder may be hydroxypropyl cellulose, and have a weight average molecular weight of less than 200,000. When hydroxypropyl cellulose with a weight average molecular weight of 200,000 or more is used, both the 5-minute and 30-minute dissolution rates are low, which is not preferable in terms of bioavailability.

Meanwhile, regarding the Carr's index used as a measure of flowability in formulation, the Carr's index of the granules is preferably 21 to 25.

In the pharmaceutical composition of the present invention, the granules may be dry granules, but the present invention is not limited thereto. In another embodiment, the granules may be wet granules.

In the present invention, the pharmaceutical composition may have a dosage form for oral administration, such as a tablet or a capsule. In one embodiment of the present invention, the pharmaceutical composition may have a tablet formulation.

In an exemplary embodiment, the pharmaceutical composition may comprise 0.3 mg of the compound of Chemical Formula 1.

The pharmaceutical composition according to the present invention may be orally administered once a day, but the present invention is not limited thereto.

### [Advantageous Effects]

The present invention relates to a pharmaceutical composition for use in preventing or treating senescence-related cardiovascular diseases, which comprises enavogliflozin as an active ingredient, and the enavogliflozin of the present invention exhibits an excellent effect on endothelial dysfunction which is caused by vascular endothelial senescence and the resulting prevention and treatment of senescence-related cardiovascular diseases.

### [Description of Drawings]

FIG. 1 is a diagram showing nanoplastics with a spherical form according to one example of the present invention.
FIG. 2 is a graph showing a zeta potential and a hydrodynamic diameter according to one example of the present invention.
FIG. 3 is a graph showing the cytotoxic result of nanoplastics according to one example of the present invention.
FIG. 4 is a diagram showing the penetration of nanoplastics with a fluorescent tag into cells according to one example of the present invention.
FIG. 5 is a diagram and graph showing the β-galactosidase (SA-β-Gal) activity of coronary artery rings exposed to nanoplastics according to one example of the present invention.
FIG. 6 is a graph showing the improvement in cell proliferation according to one example of the present invention.
FIG. 7 is a diagram and graph showing the expression of cellular senescence markers p53 and p21 according to one example of the present invention.
FIG. 8 is a diagram and graph showing the indicators of ROS production in a nanoplastic-exposed group according to one example of the present invention.
FIG. 9 is a diagram and graph showing the expression of membrane catalytic subunits of NADPH oxidase, Nox2 and p22^{phox}, with the exposure to nanoplastics according to one example of the present invention.
FIG. 10 is a graph showing the degree of vasorelaxation due to exposure to nanoplastics according to one example of the present invention.
FIG. 11 is a diagram and graph showing the expression of eNOS protein in a nanoplastic-exposed group according to one example of the present invention.

### [Modes of the Invention]

Hereinafter, one or more exemplary embodiments will be described in further detail with reference to examples. However, these examples are intended to illustrate one or more specific examples and the scope of the present invention is not limited to these examples.

### Example 1. Physicochemical properties of nanoplastics

The particle size of polystyrene nanoplastics used in an experiment is 25 nm on average, and the concentration of the polystyrene nanoplastics is 10 mg/mL (dissolved in 10% dimethyl sulfoxide (DMSO)). The shape and structure of nanoplastics were measured using a super-resolution field emission scanning electron microscope (Regulus 8230; Hitachi High-tech, Japan), and zeta potential distribution and a hydrodynamic diameter were measured using a dynamic light scattering device (Zetasizer Nano ZS90; Malvern Instruments, Malvern, UK).

### Example 2. Evaluation of cytotoxicity and intracellular penetration of nanoplastics

### 2-1. Cytotoxicity of nanoplastics

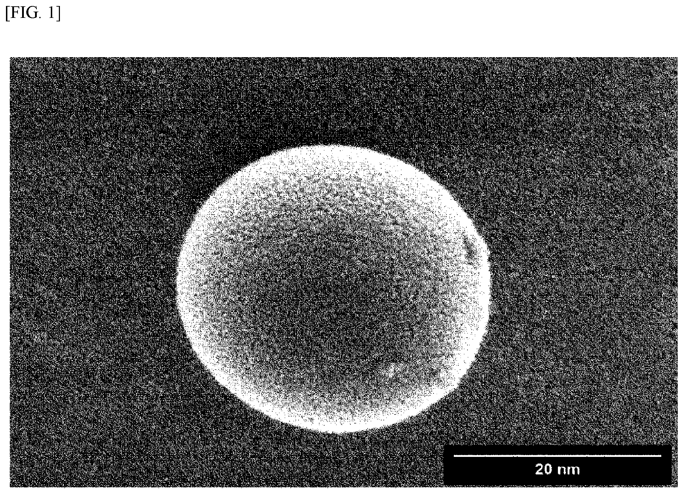

After excising a porcine coronary artery and treating it with collagenase for 20 minutes, porcine coronary artery endothelial cells were obtained by centrifugation. Subsequently, the cells were cultured in DMEM (high-glucose; GenDEPOT) containing 10% FBS, penicillin (100 U/mL), streptomycin (100 U/mL), and fungizone (250 µg/mL). The cells were seeded at 1×10⁴ cells/well in a 96-well plate to confirm the cytotoxicity of nanoplastics. Nanoplastics were treated by concentrations (0.1, 0.3, 1, 3, 10, 30, and 100 µg/mL), and incubated in the 96-well plate for 24 hours. Since the absorbance of nanoplastics was inhibited by serum (FBS), nanoplastics were treated in an FBS-free medium and then incubated for 24 hours. After the treatment with MTT (0.1 mg/mL) for 4 hours, 200 µL DMSO was added to dissolve formazan crystals. Afterward, absorbance was measured at 540 nm (Enspire Multilabel Reader; Perkin Elmer Ltd.), and the results are shown in FIGS. 1 to 3.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

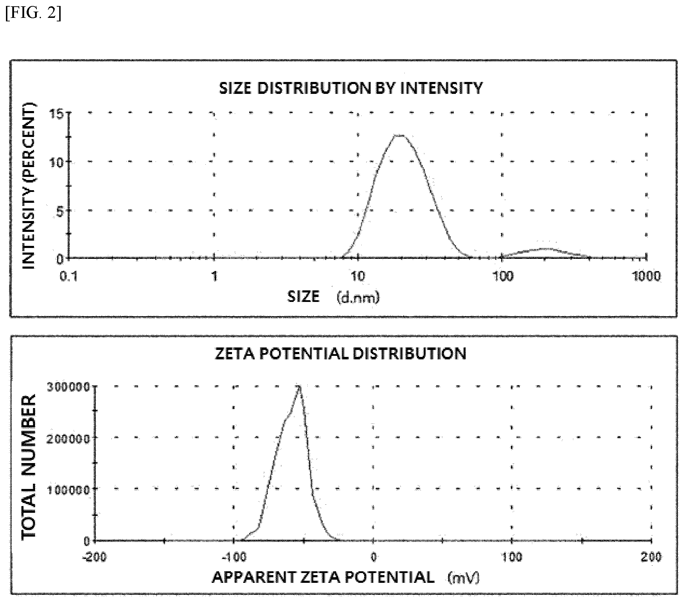

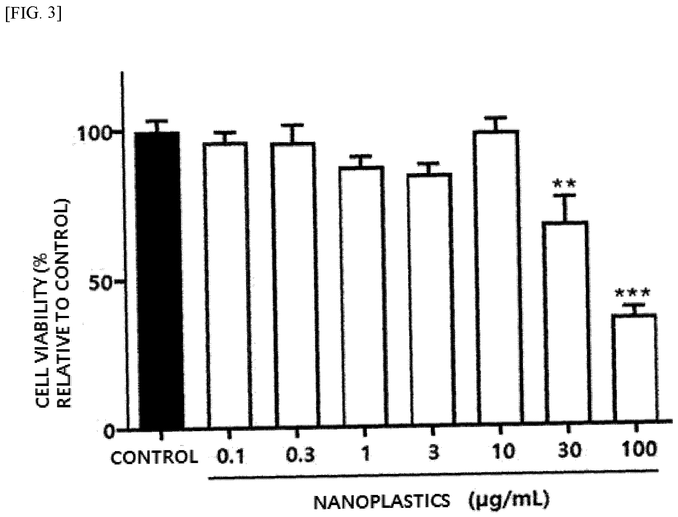

As confirmed in FIGS. 1 to 3, the shape of nanoplastics was spherical (FIG. 1), and the zeta potential and hydrodynamic diameter were confirmed to be -40.93 ± 0.58 mV and 21.97 ± 0.53 nm, respectively (FIG. 2). This indicates that nanoplastics do not aggregate in a solution. In addition, it was confirmed that the cytotoxic result of nanoplastics showed that no cytotoxicity was observed with treatment of up to 0.1 to 10 µg/mL, but cell viability was shown at 73.33% and 45.15% with treatment of 30 µg/mL and 100 µg/mL, respectively, indicating that cytotoxicity was shown (FIG. 3).

### 2-2. Intracellular penetration of nanoplastics

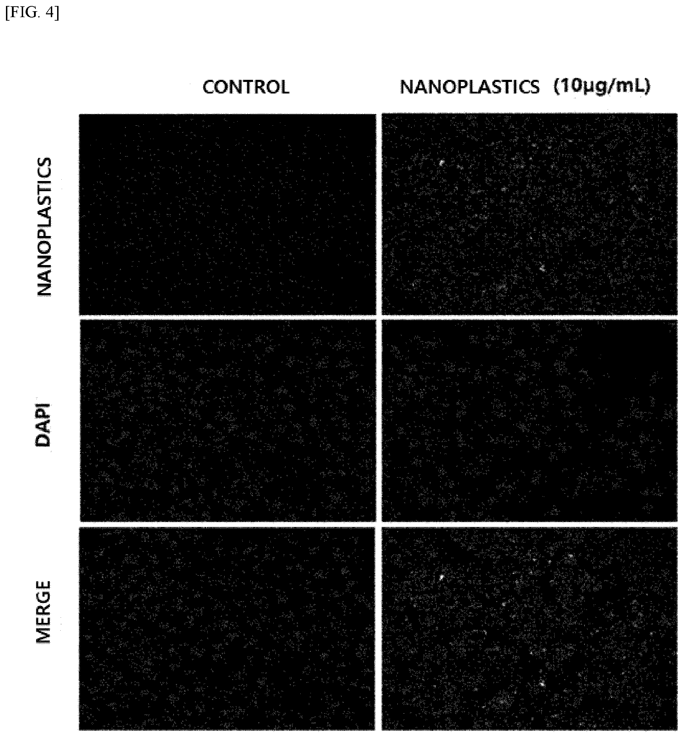

To confirm the intracellular penetration of nanoplastics, physicochemical properties were examined. After excision of the porcine coronary artery, the porcine coronary artery endothelial cells were obtained by centrifugation after treatment with collagenase for 20 minutes. Then, the cells were cultured in DMEM (high-glucose; GenDEPOT) containing 10% FBS, penicillin (100 U/mL), streptomycin (100 U/mL), and fungizone (250 µg/mL). The cells were seeded at 1×10⁵ cells/well in a 2-well Lab-Tek chamber side (Thermo Scientific, USA), and divided into a control and a fluorescent tag-attached nanoplastic (Life Technologies Corp., Eugene, Oregon) group to perform an experiment. The fluorescent tag-attached nanoplastics were treated at 10 µg/mL. After 24-hour culture, the cells were washed with phosphate buffered saline (PBS), fixed with 4% formaldehyde for 15 minutes, and then stained with 4',6-diamidino-2-phenylindole (DAPI, 10 µM). The intracellular penetration of nanoplastics was analyzed using a ZEISS LSN980 confocal microscope, and the results are shown in FIG. 4.

As confirmed in FIG. 4, it was confirmed that the fluorescent tag-attached nanoplastics penetrated into the cells.

### Example 3. Senescence-related evaluation

β-galactosidase (SA-β-Gal) activity, cell proliferation and cellular senescence markers were detected.

### 3-1. Evaluation of β-galactosidase activity

Senescence-related β-galactosidase (SA-β-Gal) activity is the characteristic of senescent cells, and used as a histochemical marker for senescence.

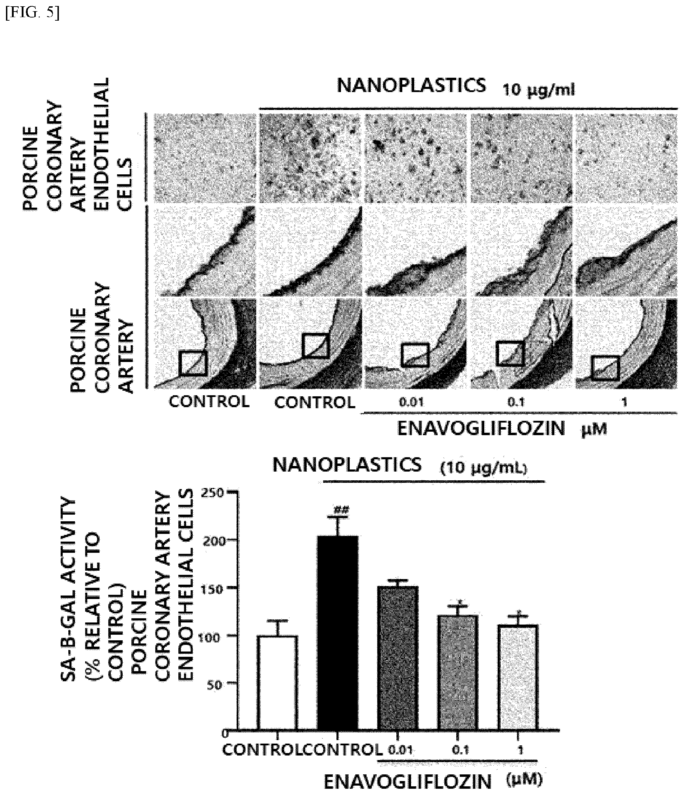

Specifically, the porcine coronary artery was cut into a size of 2 to 3 mm to form coronary artery rings. Next, the coronary artery rings were divided into a control, a group treated with 10 µg/mL of nanoplastics, and groups treated with 10 µg/mL of the nanoparticles + different concentrations of enavogliflozin (0.01, 0.1, and 1 µM) to perform the experiment. After 24-hour culture, the cells were fixed with 4% formaldehyde, and stained with an X-gal dye to allow a reaction for one day. Subsequently, the coronary artery rings were immersed in an optimal cutting temperature (OCT) solution (Leica Biosystems) and cut to 10 µm sections using a cryotome at -25 °C and observed under a microscope. The results are shown in the photograph in FIG. 5. In addition, the evaluation of β-galactosidase activity in endothelial cells derived from the porcine coronary artery is as follows. The coronary artery-derived endothelial cells were seeded at 5×10⁴ cells/well in a 12-well plate and divided into a control, and groups treated with 10 µg/mL of nanoplastics, and the nanoparticles + different concentrations of enavogliflozin (0.01, 0.1, and 1 µM) to perform the experiment. After 24-hour culture, the cells were fixed with 4% formaldehyde solution (1 mL/well) for 15 minutes, and treated with an X-gal dye (1 mg/mL) to allow a reaction for 16 hours. When X-gal was hydrolyzed by β-galactosidase activity, it turned blue, and the phenomenon by group was observed under a microscope. The results are shown graphically in FIG. 5.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 5, the β-galactosidase (SA-β-Gal) activity was increased in the coronary artery rings exposed to nanoplastics. On the other hand, in the groups treated with different concentrations of enavogliflozin (0.01, 0.1, and 1 µM), the β-galactosidase (SA-β-Gal) activity was significantly reduced.

### 3-2. Evaluation of cell proliferation effect

Cell cycle arrest is one of the main features of cellular senescence. It was confirmed whether enavogliflozin could improve decreased proliferation of endothelial cells, that is, cellular senescence, caused by the exposure to nanoplastics.

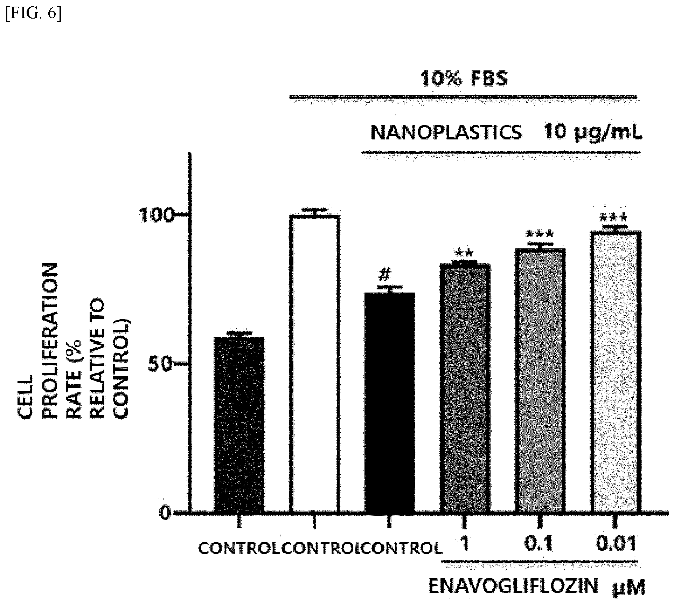

Specifically, after extracting the porcine coronary artery, the porcine coronary artery was treated with collagenase for 20 minutes and centrifuged to obtain coronary artery endothelial cells. The cells were seeded at 1×10⁴ cells/well in a 96-well plate. The cells were incubated in DMEM containing 10% FBS, penicillin (100 U/mL), streptomycin (100 U/mL), and fungizone (250 µM) in a 5% CO₂ incubator at 37 °C. The cells were divided into a control, a group treated with 10 µg/mL of nanoplastics, and groups treated with the nanoparticles + different concentrations of enavogliflozin (0.01, 0.1, and 1 µM) to perform the experiment, and the cell proliferation effect was measured using a CellTiter 96 Aqueous One Solution Cell Proliferation Assay (Promega Corporation, USA) kit. Samples were treated and incubated for 24 hours, and treated with an MTS tetrazolium solution (20 µL/well) to allow a reaction for 3 hours, and then absorbance was measured at 490 nm using an Enspire Multilabel Reader (Perkin Elmer Ltd.). The results are shown in FIG. 6.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 6, it was confirmed that cell proliferation was significantly decreased in the nanoplastic-exposed group, whereas cell proliferation was improved by enavogliflozin in the enavogliflozin-treated group.

### 3-3. Evaluation of expression of endothelial cell senescence markers

The expression of cellular senescence markers p53 and p21 was confirmed in porcine coronary artery endothelial cells.

Specifically, the cells were seeded at 1×10⁵ cells/well in a 6-well plate. The cells were cultured in DMEM containing 10% FBS, penicillin (100 U/mL), streptomycin (100 U/mL), and fungizone (250 µg/mL) in a CO₂ incubator at 37 °C. The cells were divided into a control, a group treated with 10 µg/mL of nanoplastics, and groups treated with the nanoparticles + different concentrations of enavogliflozin (0.01, 0.1, and 1 µM), and then the cells were treated with samples and incubated for 24 hours.

To lyse the porcine coronary artery endothelial cells, 1X RIPA buffer was added to each well and the cells were lysed for 10 minutes. The cells were scraped with a scraper and centrifuged at 14,000 rpm and 4 °C for 15 minutes. Protein quantification of the obtained cell lysate was measured using Bio-Rad DC protein reagent. Analysis was performed by equally loading 15 µg of protein.

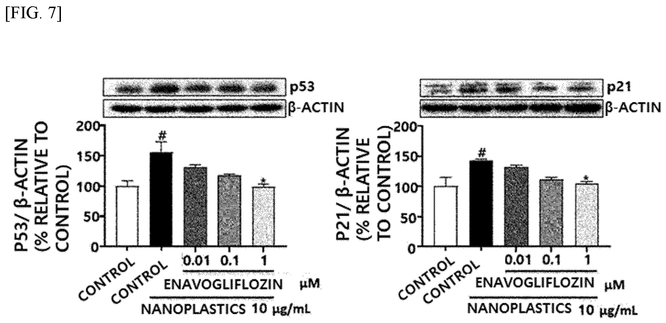

After reacting with p53 (1:1,000), p21 (1:1,000), and β-actin (1:1,000) as primary antibodies at 4 °C for 24 hours, the cells were washed 3 to 5 times with 1X TBS-T buffer for 10 minutes. Secondary antibodies were reacted at a ratio of 1:20,000 for 1 hour at room temperature, followed by washing 3 to 5 times with 1X TBS-T buffer for 10 minutes. Protein bands were identified using Amersham imager 680 after treatment with an ECL solution. The results are shown in FIG. 7.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 7, the expression of the p53 and p21 proteins was greatly increased in the nanoplastic-treated group, whereas cellular senescence marker proteins were significantly decreased in a concentration-dependent manner (0.01, 0.1, and 1 µM) in the enavogliflozin-treated group.

### Example 4. Evaluation of oxidative stress of cells

Oxidative stress was measured using dihydroethidium (DHE) and 2',7'-dichlorodihydrofluorescein diacetate (DCF-DA).

Specifically, porcine coronary artery endothelial cells were seeded at 1×10⁴ cells/well in a black 96-well plate. The cells were divided into a control, a group treated with 10 µg/mL of nanoplastics, and groups treated with 10 µg/mL of the nanoparticles + different concentrations of enavogliflozin (0.01, 0.1, and 1 µM) to perform the experiment. The cells were incubated in DMEM containing 10% FBS, penicillin (100 U/mL), streptomycin (100 U/mL), and fungizone (250 µg/mL) in a CO₂ incubator at 37 °C. After 24-hour incubation, the amount of reactive oxygen species (ROS) produced was measured by fluorescent absorbance (excitation/emission 485/535 nm).

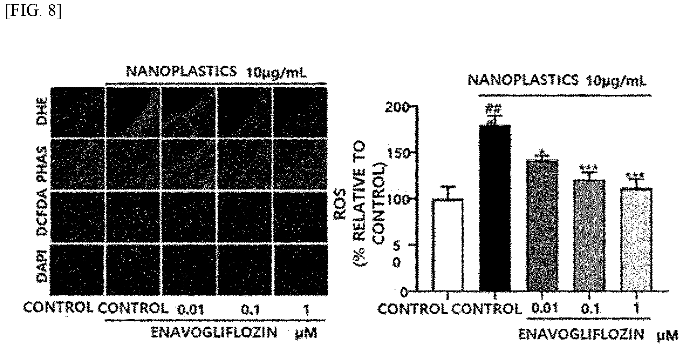

To measure the ROS produced also in the coronary artery rings, the coronary artery rings were treated with samples for each group, and immersed in a frame containing an OCT solution (Leica Biosystems) and frozen in liquid nitrogen. The frozen result was cut into 10 µm sections using a cryotome, and the sections were stained with DHE (10 µM) and incubated at 37 °C for 45 minutes, washed with PBS, and then observed using a fluorescence microscope. The results are shown in FIG. 8.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 8, it was confirmed that the fluorescence intensity of DCF-DA and DHE, which are indicators of ROS production, significantly increased in the nanoplastic-exposed group, and the fluorescence intensity significantly decreased in the groups treated with different concentrations of enavogliflozin (0.01, 0.1, and 1 µM).

### 4-2. Evaluation of expression of oxidative stress-associated markers of cells

In the porcine coronary artery endothelial cells, it is known that NADPH oxidase produces a peroxide in endothelial cells to cause cellular senescence and endothelial dysfunction. Oxidative stress in cells was assessed by measuring the expression of Nox2 and p22phox, which are membrane catalytic subunits of the NAPDH oxidase.

Specifically, the cells were seeded at 1×10⁵ cells/well in a 6-well plate. The cells were incubated in DMEM containing 10% FBS, penicillin (100 U/mL), streptomycin (100 U/mL), and fungizone (250 µg/mL) in a CO₂ incubator at 37 °C. The cells were divided into a control, a group treated with 10 µg/mL of nanoplastics, and groups treated with nanoplastics + different concentrations of enavogliflozin (0.01, 0.1, and 1 µM), treated with a sample, and then incubated for 24 hours.

To lyse the porcine coronary artery endothelial cells, 1X RIPA buffer was added to each well and the cells were lysed for 10 minutes. The cells were scraped with a scraper and centrifuged at 14,000 rpm and 4 °C for 15 minutes. Protein quantification of the obtained cell lysate was measured using Bio-Rad DC protein reagent. Analysis was performed by equally loading 15 µg of protein.

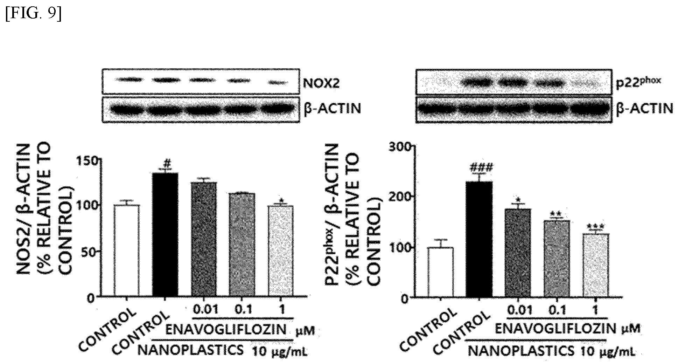

After reacting with Nox2 (1:1,000), p22phox (1:1,000), and β-actin (1:1,000) as primary antibodies at 4 °C for 24 hours, the cells were washed 3 to 5 times with 1X TBS-T buffer for 10 minutes. Secondary antibodies were reacted at a ratio of 1:20,000 for 1 hour at room temperature, followed by washing 3 to 5 times with 1X TBS-T buffer for 10 minutes. Protein bands were identified using Amersham imager 680 after treatment with an ECL solution. The results are shown in FIG. 9.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 9, Nox2 and p22^{phox}, which are the membrane catalytic subunits of NADPH oxidase, were significantly increased by the exposure to nanoplastics. On the other hand, in the groups treated with different concentrations of enavogliflozin (0.01, 0.1, and 1 µM), the related proteins were significantly decreased in a concentration-dependent manner.

Therefore, it was confirmed that enavogliflozin inhibited ROS production caused by nanoplastics and reduced oxidative stress.

### Example 5. Evaluation of vascular reactivity

### 5-1. Evaluation of vascular function

Vascular endothelial cells play an important role in maintaining vascular homeostasis, but senescence is known to cause vascular dysfunction. Therefore, vascular reactivity of vascular endothelial cells was evaluated to see whether enavogliflozin prevents vascular dysfunction caused by the exposure to nanoplastics. In addition, the evaluation was performed together with the competing material, empagliflozin, and thus their effects were compared.

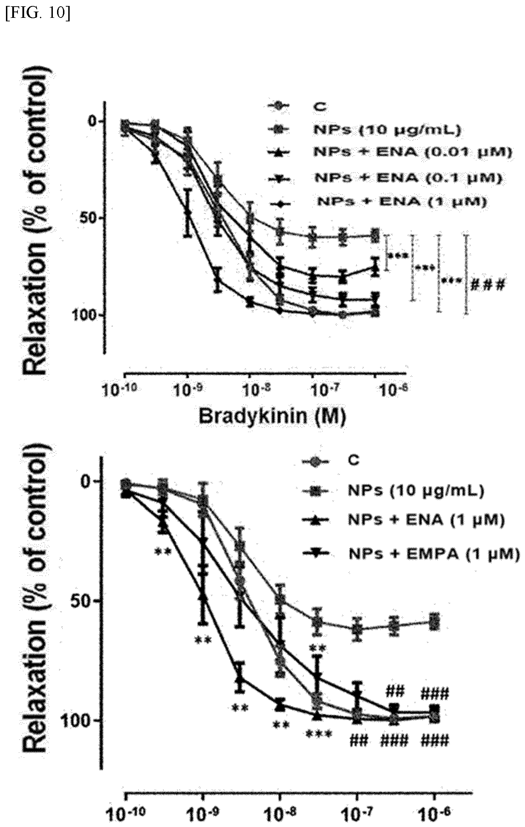

Specifically, the left anterior descending coronary artery of the porcine heart was resected into rings with a size of 2 to 3 mm. An experiment was conducted with a control, a group treated with 10 µg/mL of nanoplastics, groups treated with 10 µg/mL of nanoplastics + different concentrations of enavogliflozin (0.01, 0.1, and 1 µM), and a group treated with 10 µg/mL of nanoplastics + 1 µM empagliflozin. The coronary artery rings were placed in Crabb's solution at 37 °C in an organ chamber (95% O₂, 5% CO₂) to measure changes in isometric tension. The viability of each coronary ring was confirmed by constant isotonic loading at 5 g for 90 minutes and repeated contractions with up to 80 mM KCl. After washing for 30 minutes, the coronary artery rings were constricted to approximately 80% (maximum contraction) using the thromboxane mimetic U46619 and treated with bradykinin. The relaxation rate of blood vessels in relation to the concentration of bradykinin was then calculated. The results are shown in the graph of FIG. 10.

For statistical data analysis, Prism 5.03 software (GraphPad Software Inc., San Diego, CA, USA) was used. The test results are expressed as the mean ± standard error of the mean (SEM). The statistical method was to conduct a two-way ANOVA to verify the difference between groups, and a post hoc test for each type was performed using the verification method with the Bonferroni function. The significance level was set to be *p < 0.05.*

Bradykinin is a biomaterial, and when a bradykinin receptor is activated, endothelial nitric oxide synthase (eNOS) is activated, releasing nitric oxide (NO) and relaxing blood vessels.

As confirmed in FIG. 10, normal blood vessels that do not show vascular dysfunction are constricted by the thromboxane mimetic U46619 and relax in a concentration-dependent manner when treated with bradykinin, and almost 100% relax at a high concentration of 100 nM bradykinin (control). However, unlike the control, in the nanoplastic-treated group, even when bradykinin was added in a concentration-dependent manner, the relaxation of the coronary artery rings was reduced compared to the control, and the maximum relaxation rate did not reach 100% and still remained in a contracted state. That is, it was confirmed that endothelial dysfunction occurred due to nanoplastics. On the other hand, in the groups treated with different concentrations of enavogliflozin (0.01, 0.1, and 1 µM) together with nanoplastics, bradykinin-induced vasorelaxation was significantly increased compared to the group treated with nanoplastics alone, demonstrating that enavogliflozin improves endothelial dysfunction caused by nanoparticles (FIG. 10).

The vasorelaxation effect induced by bradykinin was compared in the groups treated with nanoplastics, and either 1 µM enavogliflozin or 1 µM empagliflozin. Compared to the group treated with nanoplastics alone, vasorelaxation was significantly increased in the group treated with enavogliflozin and nanoplastics, when bradykinin was used at 0.3 nM or more. Compared to the group treated with nanoplastics alone, vasorelaxation was significantly increased when bradykinin was used at a relatively high concentration of 30 nM or more in the group treated with empagliflozin and nanoplastics (FIG. 10).

Therefore, it was confirmed that enavogliflozin prevents endothelial dysfunction caused by the exposure to nanoplastics, and has a better effect on improving vascular dysfunction than empagliflozin.

### 5-2. Measurement of expression of eNOS

Reduced eNOS expression is a common feature in endothelial senescence, and is known to cause endothelial dysfunction. Here, the expression of eNOS was measured, thereby confirming that enavogliflozin improves the damage to the endothelial function caused by the exposure to nanoplastics.

Specifically, the cells were seeded at 1×10⁵ cells/well in a 6-well plate. The cells were cultured in DMEM containing 10% FBS, penicillin (100 U/mL), streptomycin (100 U/mL), and fungizone (250 µg/mL) in a CO₂ incubator at 37 °C. The cells were divided into a control, a group treated with 10 µg/mL of nanoplastics, and groups treated with the nanoparticles + different concentrations of enavogliflozin (0.01, 0.1, and 1 µM), and then the cells were treated with samples and incubated for 24 hours.

To lyse the porcine coronary artery endothelial cells, 1X RIPA buffer was added to each well and the cells were lysed for 10 minutes. The cells were scraped with a scraper and centrifuged at 14,000 rpm and 4 °C for 15 minutes. Protein quantification of the obtained cell lysate was measured using Bio-Rad DC protein reagent. Analysis was performed by equally loading 15 µg of protein.

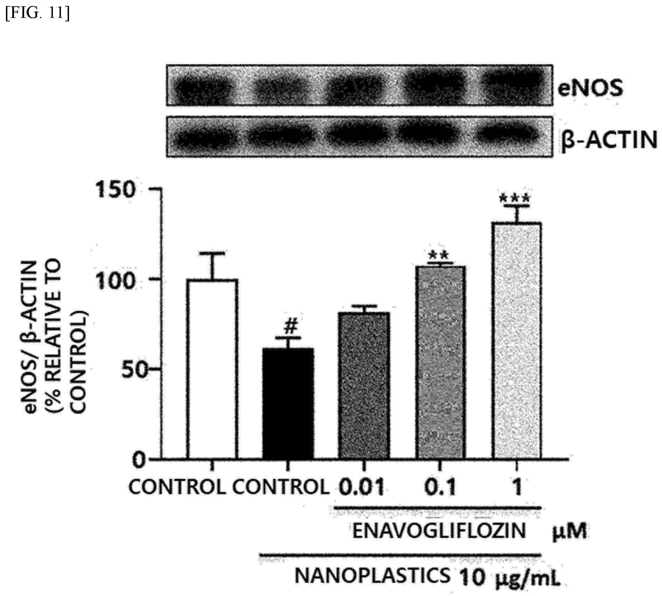

After eNOS (1:1,000) and β-actin (1:1,000) as primary antibodies were reacted at 4 °C for 24 hours, the resulting product was washed 3 to 5 times with 1X TBS-T buffer for 10 minutes. Secondary antibodies were reacted in a ratio of 1:20,000 at room temperature for 1 hour, and then the resulting product was washed 3 to 5 times with 1X TBS-T buffer for 10 minutes. Protein bands were treated with an ECL solution and detected using Amersham imager 680. The results are shown in FIG. 11.

For statistical data analysis, Prism 5.03 software (GraphPad Software Inc., San Diego, CA, USA) was used. The test results are expressed as the mean ± standard error of the mean (SEM). The statistical method was to conduct a one-way ANOVA to verify the difference between groups, and a post hoc test for each type was performed using the verification method with the Tukey function. The significance level was set to be *p < 0.05.*

As confirmed in FIG. 11, it was confirmed that eNOS protein expression was significantly reduced in the nanoplastic-exposed group, whereas enavogliflozin allows eNOS expression to significantly increase a concentration-dependent manner (0.01, 0.1, and 1 µM).

## Claims

1. A pharmaceutical composition for use in preventing or treating senescence-related cardiovascular diseases, comprising enavogliflozin as an active ingredient.

2. The composition of claim 1, wherein the senescence-related cardiovascular diseases comprise one or more selected from the group consisting of hypertrophic obstructive cardiomyopathy, severe obstructive coronary artery disease, aortic stenosis, hemodynamically significant aortic or mitral stenosis, aortic aneurysm, coronary artery disease, peripheral artery disease, and carotid artery disease.

3. The composition of claim 1, wherein the pharmaceutical composition is formulated for oral or parenteral administration.

4. The composition of claim 1, wherein the daily dose of enavogliflozin is 0.1 to 0.5 mg.

5. The composition of claim 1, which is administered once a day.
